# EUROPEAN PATENT APPLICATION

(11) **EP 3 832 377 A1**
(43) Date of publication of application: **09.06.2021**
(21) Application number: 19306596.8
(22) Date of filing: 06.12.2019
(51) Int. Cl.: G02C 1/00, G02C 5/12, A61B 3/04, G02C 13/00

(54) **METHOD AND SYSTEM FOR DETERMINING A PRESCRIPTION FOR AN EYE OF A PERSON**

(71) Applicant: ESSILOR INTERNATIONAL, 94220 Charenton-Le-Pont (FR)
(72) Inventor: LE GALL, Mathieu, 75009 PARIS (FR); FREZAL, Thibaut, 75009 PARIS (FR); FRARIER, Damien, 75009 PARIS (FR)
(74) Representative: Cabinet Novitech

(57) **Abstract**

The invention relates to an eyewear (10) comprising:
- a frame headwear (12) configured to be worn by a user, the frame headwear comprising a front part (16), and
- at least one lens holder (14), each lens holder being configured to be removably fastened to the frame headwear in a predetermined mounted position and each lens holder comprising an associated pair of ophthalmic lenses attached to said lens holder, each lens (22, 24) of the associated pair of ophthalmic lenses being configured to be movable relative to the lens holder while being attached to said lens holder.

## Description

### FIELD OF THE INVENTION

The invention relates to an eyewear. The invention further relates to a method for providing an eyeglass adapted to a user and to a method for comparing a first lens and a second lens using such eyewear.

### BACKGROUND OF THE INVENTION

Ophthalmic lenses intended to be held in a frame usually involve a prescription. The ophthalmic prescription can include a positive or negative power prescription as well as an astigmatism prescription. These prescriptions correspond to corrections enabling the wearer of the lenses to correct defects of his vision. A lens is fitted in the frame in accordance with the prescription and with the position of the wearer's eyes relative to the frame.

Lenses suitable for presbyopic wearers are multifocal lenses or progressive addition lenses, wherein the value of the power correction is different for far vision and near vision, due to the difficulties of accommodation in near vision. For example progressive addition lenses are generally determined by optimization, based on a certain number of constraints imposed on the different features of the lens.

Nevertheless, some wearer may have difficulties to adapt to the use of progressive addition lenses despite normal binocular vision and other normal clinical findings.

It is not usual for an eye care practitioner to be able to demonstrate different type of lenses. A few demonstrators exists proposing to switch the lens from a demonstrator headwear to present different type of lens, or different type of optical designs for multifocal lenses. However to this end, each trial lens is placed independently in front of an eye of the wearer and thus the fitting position is to be done each time one change lens set/type.

In addition, the comparison between different lenses in-store needs quick switching between lenses.

Thus, there is a need for clients to experiment and compare different lenses benefits by quickly and efficiently switching between the lenses.

One object of the present invention is to provide a demonstration tool to support lens sales in-store allowing to switch together lenses while maintaining client's fittings parameters for another try.

### SUMMARY OF THE INVENTION

To this end, the invention proposes an eyewear comprising:
- a frame headwear configured to be worn by a user, the frame headwear comprising a front part, and
- at least one lens holder, each lens holder being configured to be removably fastened to the frame headwear in a predetermined mounted position and each lens holder comprising an associated pair of ophthalmic lenses attached to said lens holder, each lens of the associated pair of ophthalmic lenses being configured to be movable relative to the lens holder while being attached to said lens holder.

Advantageously, such eyewear allows quickly switching two lens holders while maintaining the fitting's parameters of a client thus allowing an efficient switch between the lenses to be tested, in particular when the frame headwear is worn by the wearer.

Indeed, thanks to the eyewear, the pupillary distance and the fitting height adjustments are set once for all the tested lens holders.

Thus, such eyewear ensures the client to efficiently experiment and compare different lenses benefits.

The eyewear satisfies rapidity requirement and less eye care practitioner's settings and manipulation on the client for the test.

According to further embodiments which can be considered alone or in combination:
- each lens of the pair of ophthalmic lenses is movable relative to the associated lens holder along an axis corresponding to a nasal-temporal axis of the user when the eyewear is worn by the user such that the associated pair of ophthalmic lenses is positioned in front of the eyes of the user;
- the or each each lens holder is magnetically fastened to the frame headwear;
- the lens holder comprises two circles jointed by a bridge, the lenses being positioned within the circles ;
- a position of a nosepad of the frame headwear relative to the frame headwear is adjustable vertically when the eyewear is worn by the user;
- a position of the lens holder relative to the frame headwear is adjustable vertically when the eyewear is worn by the user such that the associated pair of ophthalmic lenses is positioned in front of the eyes of the user;
- the eyewear comprises a set of at least two lens holders, each pair of ophthalmic lenses of the set comprises a right lens and a left lens, the right lenses of the set having same right contour and optical centers of the right lenses being positioned in same place relative to the right contour and the left lenses of the set having same left contour and optical centers of the left lenses being positioned in same place relative to the left contour;
- the eyewear comprises a set of at least two lens holders, each pair of ophthalmic lenses being different from one lens holder to another of the set, preferentially having different optical powers and/or different optical designs from one lens holder to another;
- the set of at least one lens holder comprises:
   ∘ a first subset of at least one first lens holder, each first lens holder having an associated pair of single vision lenses with a first optical power, and
   ∘ a second subset of at least one second lens holder, each second lens holder having an associated pair of progressive or bifocal addition lenses with at least one zone having the first power;
- the single vision lenses of the first subset have a first optical power and the progressive addition lenses of the second subset have an addition of same dioptric value as the first optical power;
- the set of at least one lens holder comprises:
   ∘ a first subset of at least one first lens holder, each first lens holder having an associated pair of single vision lenses having at least a first optical power, and
   ∘ a second subset of at least one second lens holder, each second lens holder having an associated pair of bifocal lenses or trifocal lenses having at least one optical power equal to the first optical power;
- the set of at least one lens holder comprises:
   ∘ a first subset comprising at least a first lens holder having an associated pair of single vision lenses having a first optical power, and a second lens holder having an associated pair of single vision lenses having a second optical power distinct from the first optical power, and
   ∘ a second subset comprising at least a third lens holder, the third lens holder having an associated pair of third lenses, the third lenses comprising at least a near vision zone having an optical power equal to the first optical power and a far vision zone having an optical power equal to the second optical power, the third lenses being progressive additional lenses or bifocal lenses or trifocal lenses;
- the lenses have different optical designs and/or different optical powers and/or different tint.

Another object of the invention is directed to a method for providing an eyewear to a user using an eyewear comprising a frame headwear configured to be worn by a user, the frame headwear comprising a front part, and at least one lens holder, each lens holder being configured to be removably fastened to the frame headwear in a predetermined mounted position and each lens holder comprising an associated pair of ophthalmic lenses attached to said lens holder, each lens of the associated pair of ophthalmic lenses being configured to be movable relative to the lens holder while being attached to said lens holder, the method comprising at least:
- providing the frame headwear;
- providing a lens holder;
- fastening the lens holder to the frame headwear;
- for each lens of the lens holder, moving the position of the lens relative to the lens holder to adjust the position of the lens to the pupillary distance of the user.

According to further embodiments of the method which can be considered alone or in combination:
- the method further comprises moving vertically the position of the lens holder relative to the frame headwear or vertically a position of a nosepad of the frame headwear relative to the frame headwear to align a pupil center of an eye of the user with a landmark of a lens placed in front of the eye;
- the eyewear comprises a set of at least two lens holders and the method further comprises:
   ∘ providing another lens holder of the set;
   ∘ switching the lens holder with the other lens holder;
   ∘ for each lens of the other lens holder, moving the position of the lens relative to the other lens holder to adjust the position of the lens of the other lens holder to the pupillary distance of the user;
- when the user wear spectacles, the method further comprises:
   ∘ mounting the frame headwear over the spectacles, and
   ∘ switching in and/or out the lens holder over the spectacles.

Another object of the invention is directed to a method for comparing two different lenses, the method comprising at least:
- providing a frame headwear;
- providing a first lens holder and a second lens holder,
- for each lens holder of the first lens holder and a second lens holder:
   ∘ fastening the lens holder to the frame headwear;
   ∘ for each lens of the lens holder, moving the position of the lens relative to the lens holder to adjust the position of the lens to the pupillary distance of the user,
- switching from the first lens holder to the second lens holder at least once after adjusting the position of the lens to the pupillary distance of the user.

According to an embodiment, the lenses have different optical designs and/or different optical powers and/or different tint.

### BRIEF DESCRIPTION OF THE DRAWINGS

Embodiments of the invention will now be described, by way of example only, and with reference to the following drawings in which:
- Figures 1 and 2 are schematic representations of an eyewear according to an embodiment of the invention; in figure 1, the eyewear is assembled contrary to figure 2;
- Figure 3 is a schematic representations of a lens holder of the eyewear illustrated on figures 1 and 2;
- Figure 4 is an illustration of a part of the lens holder of figure 3 comprising a right lens;
- Figures 5 and 6 illustrates respectively a wrong and a right setting of the fitting height of the eyewear with respect to the wearer's eyes;
- Figure 7 is an illustration of a chart-flow of a method for providing an eyeglass adapted to a user using an eyewear according to the invention; and
- Figure 8 is an illustration of a chart-flow of a method or comparing a first lens and a second lens according to an embodiment of the invention.

Elements in the figures are illustrated for simplicity and clarity and have not necessarily been drawn to scale. For example, the dimensions of some of the elements in the figure may be exaggerated relative to other elements to help improve the understanding of the embodiments of the present invention.

### DETAILED DESCRIPTION OF PREFERRED EMBODIMENTS

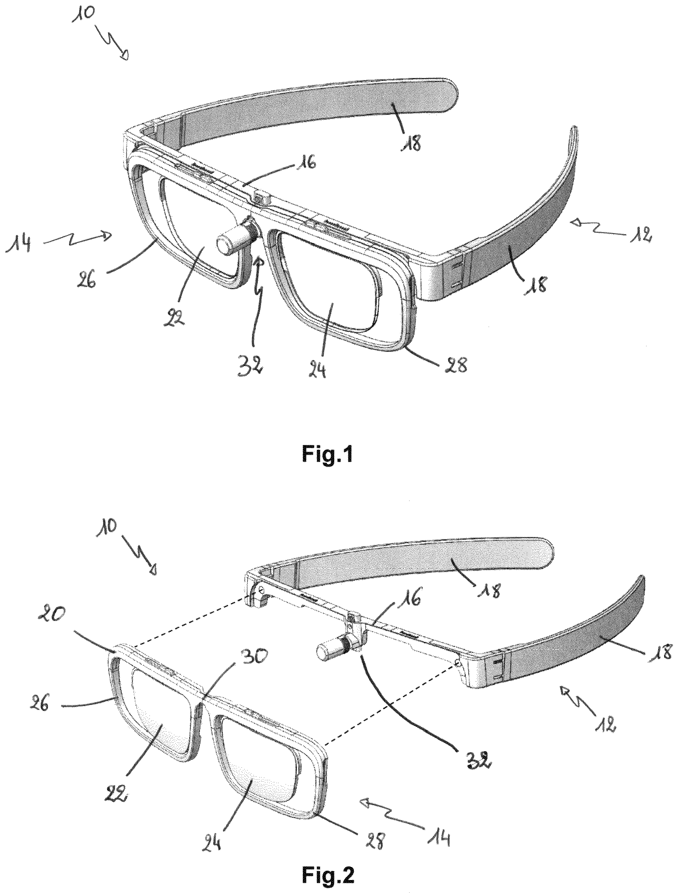

The invention relates to an eyewear comprising a frame headwear and at least one lens holder. Figures 1 and 2 represent an example of eyewear 10 comprising a frame headwear 12 and a lens holder 14. In figure 1, the eyewear is assembled contrary to figure 2 wherein the lens holder 14 is not yet fastened to the frame headwear 12.

The frame headwear 12 is configured to be worn by a user. To this end, the frame headwear 12 comprises a front part 16, a first and second temples 18. The first and second temples 18 are configured to be attached to the front part 16, for example using hinges and screws. The frame headwear is defined as being part of a frame that doesn't bear any lenses while the lens holder is not fastened to it.

Each lens holder 14 is configured to be removably fastened to the frame headwear 12 in a predetermined mounted position.

For example, the lens holder may be advantageously magnetically fastened to the frame headwear allowing a quick, repeatable and reliable positioning of the lens holder relative to the frame headwear 12. Two lens holders can thus be easily switched with repeatable replacement on the frame headwear.

The frame headwear fastened with the lens holder may be used as an eyeglass frame by the user or may be positioned by the wearer over spectacles already worn by the user. To this end, the frame headwear is configured to be mounted over possible spectacles of the user.

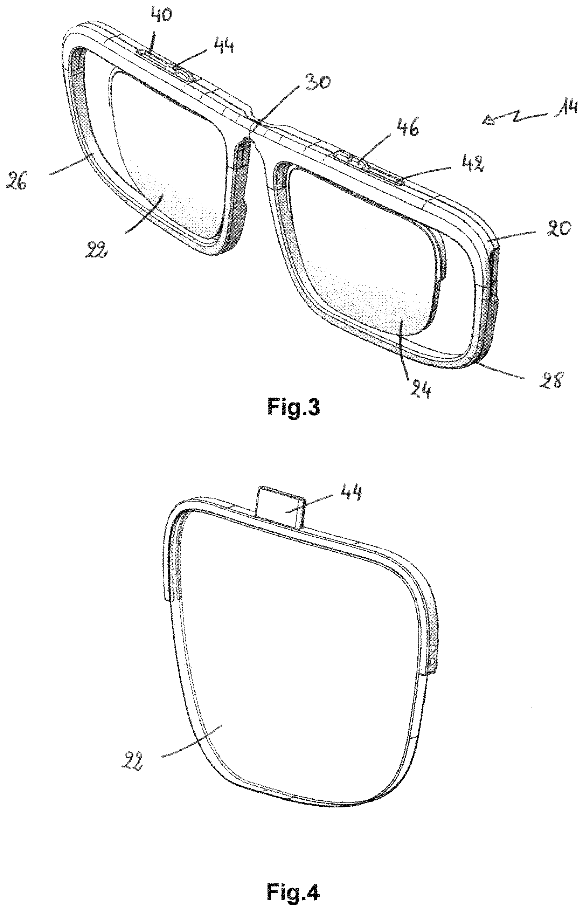

The frame headwear 12 further comprises a nose pad 32 arranged to rest on the nose of the user when the eyewear 10 in worn by the user. With reference to figure 3, the lens holder 14 comprises a frame 20 and an associated pair of optical lenses attached to said lens holder 14 via the frame 20. The associated pair of optical lenses comprises a first optical lens 22 and a second optical lens 24. The first and second optical lenses 22, 24 are preferably ophthalmic lenses. The first optical lens 22 and a second optical lens 24 are coupled to a lens holder ensuring an efficient switching between two lens holders in particular when the frame headwear is worn by the wearer..

Advantageously, the frame 20 of the lens holder 14 comprises a first circle 26 and a second circle 28 linked by a bridge 30 allowing to handle the lens holder quickly without putting fingers on the lenses when switching two lens holders. Indeed, this enables to preserve the lens from being moved relative to the lens holder during handling and from being soiled during the quick switching, which would be detrimental to the quality of the comparison. Each of the first and second circles 26, 28 comprises frame rims arranged to receive respectively the first and second lenses 22, 24 such that the lenses 22, 24 are positioned within a corresponding circle 26, 28.

The first lens 22 and the second lens 24 are arranged in order to be placed in front of respectively a right eye and a left eye (not illustrated on figures 1 to 3) of the user when the lens holder 14 is attached to the frame headwear 12 and the eyewear 10 in worn by the user.

The first lens 22 and the second lens 24 are each configured to be movable independently of one another relative to the lens holder 14 while being attached to said lens holder 14. Advantageously, each lens 22, 24 of the pair is movable relative to the lens holder along an axis corresponding to a nasal-temporal axis of the user when the eyewear is worn by the user such that the associated pair of ophthalmic lenses is positioned in front of the eyes of the user.

In other words, the lens holder comprises means for adjusting the position of each lens 22, 24 along an axis corresponding to a nasal-temporal axis of the user when the eyewear is worn allowing to adjust the pupillary distance independently for each eye. For example, a first slide 40 and a second slide 42 are arranged in the frame 20 of the lens holder and each first lens 22 and second lens 24 comprises respectively a first complementary mean 44 and a second complementary mean 46, as illustrated on figure 4. The first complementary mean 44 is arranged and configured to be inserted in the first slide 40 such that when an eye care practitioner slides the first complementary mean 44 in the first slide 40, the first lens 22 moves relative to the lens holder along an axis corresponding to the nasal-temporal axis of the user allowing the setting of the pupillary distance for the right eye.

In the same manner, the second complementary mean 46 is arranged and configured to be inserted in the second slide 42 such that when the eye care practitioner slides the second complementary mean 46 in the second slide 42, the first lens 22 moves relative to the lens holder along an axis corresponding to the nasal-temporal axis of the user allowing the setting of the pupillary distance for the left eye independently of the setting of the pupillary distance for the right eye.

Advantageously, a position of the frame headwear 12 is further adjustable vertically relative to the user when the eyewear is worn by the user such that the associated pair of ophthalmic lenses is well positioned in front of the eyes of the user while the lens holder 14 is attached to the frame headwear 12.

In other words, the frame headwear comprises means for adjusting the position of the nosepad 32 of the frame headwear 12 relative to the frame headwear 12 along a vertical axis when the eyewear is worn by the user allowing the setting of the fitting height. Thanks to the invention, the fitting height is set once for a lens holder and is then maintained for other lens holders.

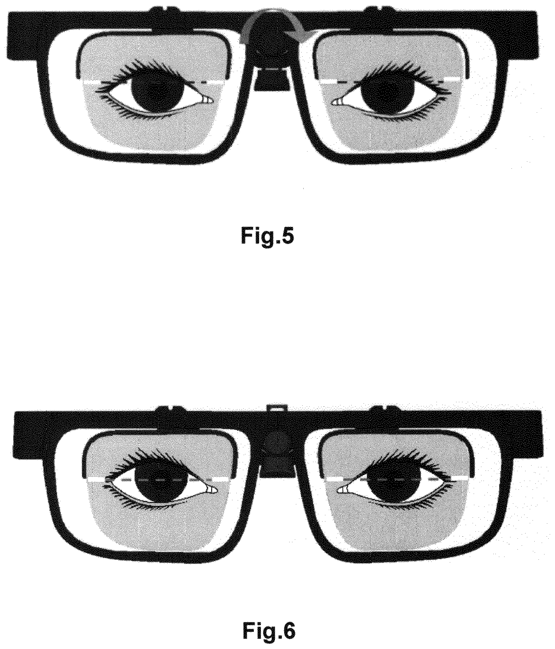

For example, the lenses 22, 24 comprises landmarks and the frame headwear 14 comprises a system for moving vertically the position of the frame headwear 12 relative to the user allowing the alignment of the pupil centers of the eyes of the user with the landmarks of the lenses 22, 24 as illustrated on figures 5 and 6. Figure 5 illustrates that the fitting height of the eyewear is misaligned with respect to the wearer's eyes while in figure 6, the setting of the fitting height is correct.

For example, the system for moving vertically the position of the nosepad 32 of the frame headwear 12 relative to the frame headwear 12 when the eyewear is worn may comprise a rack and a pinion configured to cooperate with the rack.

According to an embodiment, a position of the lens holder 14 relative to the frame headwear 12 may further be adjustable vertically when the eyewear is worn by the user such that the associated pair of ophthalmic lenses is positioned in front of the eyes of the user.

According to a preferred embodiment, the eyewear 10 comprises a set of a plurality of lens holders. Each lens holder of the set is configured to be removably fastened to the frame headwear in the same manner and as described hereinbefore and each lens of the associated pair of ophthalmic lenses is also movable relative to the lens holder as described hereinbefore.

Each pair of ophthalmic lenses of the set comprises a right lens 22 and a left lens 24 intended to be respectively placed in front of the right eye and the left eye of the user when wearing the eyewear.

The right lenses of the set have same right contour and optical centers of the right lenses are positioned in same place relative to the right contour. In the same manner, the left lenses of the set have same left contour and optical centers of the left lenses are positioned in same place relative to the left contour

Each pair of ophthalmic lenses is different from one lens holder to another of the set allowing the comparison of the benefits of the lens holders of the set. In other words, at least one among the right lens and the left lens of a lens holder is different from one lens holder to another of the set.

According to a preferred embodiment, the pairs of ophthalmic lenses of the set have different optical powers and/or different optical designs from one lens holder to another.

In the sense of the invention an "optical design" designates the set of parameters that allows defining the surfaces of an ophthalmic lens. For example, it may comprise the surface equation, position and orientation of a surface of a face of an ophthalmic lens, such equation, position and orientation being defined in a coordinate system.

According to a first embodiment, the set of lens holders comprises a first subset of at least one first lens holder, and a second subset of at least one second lens holder.

Each first lens holder has an associated pair of single vision lenses and each second lens holder has an associated pair of progressive addition lenses.

Preferably, at least one single vision lens of the first subset has a first optical power and at least one progressive addition lens of the second subset has at least one zone having the first power or an addition of same dioptric value as the first optical power.

Advantageously, such eyewear with such set of lens holders according to the first embodiment allows the client to efficiently experiment and compare progressive addition lens benefits relative to those of the single vision lens by quickly switching the corresponding lens holders while maintaining the fitting's parameters of the client in particular when the frame headwear is worn by the user.

According to a second embodiment, each first lens holder of the first subset has an associated pair of single vision lenses and each second lens holder of the second subset has an associated pair of bifocal lenses or trifocal lenses.

Preferably, at least one single vision lens of the first subset has a first optical power and at least one bifocal or trifocal lens of the second subset has at least one zone having the first power.

Advantageously, such eyewear with such set of lens holders according to the second embodiment allows the client to efficiently experiment and compare bifocal or trifocal lens benefits relative to those of the single vision lens by quickly switching the corresponding lens holders while maintaining the fitting's parameters of the client in particular when the frame headwear is worn by the user.

According to a third embodiment, the set of at least one lens holder comprises a first subset comprising at least a first lens holder and a second lens holder and a second subset comprising at least a third lens holder.

The first lens holder comprises an associated pair of single vision lenses having a first optical power P1, and the second lens holder comprises an associated pair of single vision lenses having a second optical power P2 distinct from the first optical power P1.

Preferably, at least one third lens holder comprises an associated pair of third lenses comprising at least a near vision zone having an optical power equal to the first optical power P1 and a far vision zone having an optical power equal to the second optical power P2.

Of course, the third lenses may be progressive additional lenses, bifocal lenses or trifocal lenses.

Advantageously, such eyewear with such set of lens holders according to the third embodiment allows the client to efficiently experiment and compare benefits of different lenses: progressive addition lenses, bifocal lenses, trifocal lenses and single vision lenses, by quickly switching the corresponding lens holders while maintaining the fitting's parameters of the client in particular when the frame headwear is worn by the client.

Of course all these embodiments are compatible and the lenses may have different optical designs and/or different optical powers and/or different tint and/or different functional coatings allowing an easily comparison of their benefits by a client. A set may comprise different subsets for single vision lenses, progressive addition lenses, bifocal lenses and/or trifocal lenses. For example, a set of lens holders may comprise:
- a first subset of first lens holders, each first lens holder having an associated pair of single vision lenses with a first optical power, the first optical power may be different from one single vision lens to another,
- a second subset of second lens holders, each second lens holder having an associated pair of progressive addition lenses having a near vision zone with a near vision optical power and/or a far vision zone with a far vision optical power such that at least one of the near vision optical power and the far vision power is equal to at least one first optical power of single vision lenses of a first lens holder, and/or
- a third subset of third lens holders, each third lens holder having an associated pair of bifocal or trifocal lenses having a near vision zone with a near vision optical power and/or a far vision zone with a far vision optical power such that at least one of the near vision optical power and the far vision power is equal to at least one first optical power of single vision lenses of a first lens holder.

Another object of the invention relates to a method for providing an eyeglass adapted to a user using an eyewear according to the invention and as described hereinbefore.

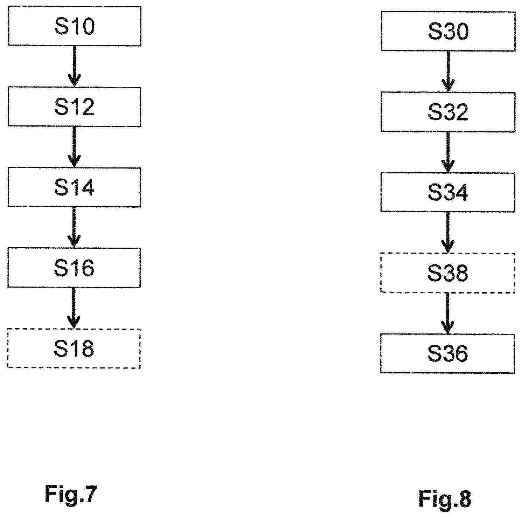

With reference to figure 7, the method comprises at least:
- providing S10 the frame headwear;
- providing S12 a lens holder;
- fastening S14 the lens holder to the frame headwear;
- for each first and second lenses of the lens holder when the eyewear is worn by the user, moving S16 the position of the lens relative to the lens holder to adjust the position of each lens to the pupillary distance of the eyes of the user.

Advantageously, the method further comprises moving S18 vertically the position of the nosepad of the frame headwear relative to the frame headwear and/or moving vertically the position of the lens holder relative to the frame headwear to align a pupil center of an eye of the user with a landmark of a lens placed in front of the eye allowing the setting of the fitting height of the eyewear to the user.

According to a preferred embodiment, the eyewear comprises a set of lens holders as disclosed hereinbefore. Each lens holder of the set comprises a pair of optical lenses. Thus, the method may further comprise:
- providing another lens holder of the set;
- switching the lens holder with the other lens holder;
- for each lens of the other lens holder, moving the position of the lens relative to the other lens holder to adjust the position of the lens of the other lens holder to the pupillary distance of the user.

Thanks to the invention, the fitting height is set once for a lens holder and is then maintained for other lens holders.

In addition, the eyewear allows the switching of both lenses at the same time and the switching between various lenses is very quick and thus efficient for a successful experience and comparison between the presented lenses in particular when the frame headwear is worn by the wearer.

According to another embodiment compatible with the previous ones, when the user already wears spectacles, the method further comprises mounting the frame headwear over the spectacles, and switching the lens holder on the spectacles by unfastening the lens holder from the frame headwear allowing an efficient comparison between the user's spectacles alone and the user's spectacles with the presented lenses. For example, if the user wears unifocal lenses having a correction in power and cylinder, the presented lenses can have an additional function that may be optical (addition for example) or complementary nature (a treatment, a hue, polarization...).

Another object of the invention relates to a method for comparing a first lens and a second lens, for example adapted to be placed in front of a same eye. The method comprises at least:
- providing S30 a frame headwear;
- providing S32 a first lens holder and a second lens holder. The first lens holder comprises at least the first lens arranged to be placed in front of the corresponding eye when the eyewear is worn. The second lens holder comprises at least the second lens arranged to be placed in front of the same eye when the eyewear is worn.

- for each lens holder of the first lens holder and the second lens holder:
   ∘ fastening the lens holder to the frame headwear;
   ∘ for each lens of the lens holder, moving S34 the position of the lens relative to the lens holder to adjust the position of the lens to the pupillary distance of the eye of the user,
- switching S36 from the first lens holder to the second lens holder at least once after adjusting the position of the lens to the pupillary distance of the user in particular when the frame headwear is worn by the wearer.

Preferably the method further comprises moving S38 vertically the position of the frame headwear relative to the user to align a pupil center of an eye of the user with a landmark of a lens placed in front of the eye allowing the setting of the fitting height of the eyewear to the user before the switching from the first lens holder to the second lens holder.

Such method and eyewear promote the detection of differences between the first lens and the second lens.

The invention as disclosed hereinbefore has the advantage to provide a demonstration tool to support lens sales in-store allowing to switch together lenses while maintaining client's fittings parameters for another try.

Advantageously, such eyewear with a plurality of lens holders allows the client to efficiently experiment and compare benefits of different lenses: progressive addition lenses, bifocal lenses, trifocal lenses and single vision lenses, by quickly switching the corresponding lens holders while maintaining the fitting's parameters of the client in particular when the frame headwear is worn by the client.

The lenses may have different optical designs and/or different optical powers and/or different tint and/or different functional coatings allowing an easily comparison of their benefits by a client.

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. An eyewear comprising:
- a frame headwear configured to be worn by a user, the frame headwear comprising a front part, and
- at least one lens holder, each lens holder being configured to be removably fastened to the frame headwear in a predetermined mounted position and each lens holder comprising an associated pair of ophthalmic lenses attached to said lens holder, each lens of the associated pair of ophthalmic lenses being configured to be movable relative to the lens holder while being attached to said lens holder.

2. The eyewear according to claim 1, wherein each lens of the pair of ophthalmic lenses is movable relative to the associated lens holder along an axis corresponding to a nasal-temporal axis of the user when the eyewear is worn by the user such that the associated pair of ophthalmic lenses is positioned in front of the eyes of the user.

3. The eyewear according to claim 1 or 2, wherein the each lens holder is magnetically fastened to the frame headwear.

4. The eyewear according to any of the preceding claims 1 to 3, wherein the lens holder comprises two circles jointed by a bridge, the lenses being positioned within the circles.

5. The eyewear according to any of the preceding claims 1 to 4, wherein a position of a nosepad of the frame headwear relative to the frame headwear is adjustable vertically when the eyewear is worn by the user and/or a position of the lens holder relative to the frame headwear is adjustable vertically when the eyewear is worn by the user such that the associated pair of ophthalmic lenses is positioned in front of the eyes of the user.

6. The eyewear according to any of the preceding claims 1 to 5, wherein the eyewear comprises a set of at least two lens holders, each pair of ophthalmic lenses of the set comprises a right lens and a left lens, the right lenses of the set having same right contour and optical centers of the right lenses being positioned in same place relative to the right contour and the left lenses of the set having same left contour and optical centers of the left lenses being positioned in same place relative to the left contour.

7. The eyewear according to any of the preceding claims 1 to 6, wherein the eyewear comprises a set of at least two lens holders, each pair of ophthalmic lenses being different from one lens holder to another of the set, preferentially having different optical powers and/or different optical designs from one lens holder to another.

8. The eyewear according to any of the preceding claims 6 to 7, wherein the set of at least one lens holder comprises:
- a first subset of at least one first lens holder, each first lens holder having an associated pair of single vision lenses with a first optical power, and
- a second subset of at least one second lens holder, each second lens holder having an associated pair of progressive or bifocal addition lenses with at least one zone having the first power.

9. The eyewear according to any of the preceding claims 6 to 7, wherein the set of at least one lens holder comprises:
- a first subset comprising at least a first lens holder having an associated pair of single vision lenses having a first optical power, and a second lens holder having an associated pair of single vision lenses having a second optical power distinct from the first optical power, and
- a second subset comprising at least a third lens holder, the third lens holder having an associated pair of third lenses, the third lenses comprising at least a near vision zone having an optical power equal to the first optical power and a far vision zone having an optical power equal to the second optical power, the third lenses being progressive additional lenses or bifocal lenses or trifocal lenses.

10. Method for providing an eyewear to a user using an eyewear comprising a frame headwear configured to be worn by a user, the frame headwear comprising a front part, and at least one lens holder, each lens holder being configured to be removably fastened to the frame headwear in a predetermined mounted position and each lens holder comprising an associated pair of ophthalmic lenses attached to said lens holder, each lens of the associated pair of ophthalmic lenses being configured to be movable relative to the lens holder while being attached to said lens holder, the method comprising at least:
- providing the frame headwear;
- providing a lens holder;
- fastening the lens holder to the frame headwear;
- for each lens of the lens holder, moving the position of the lens relative to the lens holder to adjust the position of the lens to the pupillary distance of the user.

11. Method for providing an eyewear to a user according to claim 10, wherein the method further comprises:
- moving vertically the position of the lens holder relative to the frame headwear or vertically a position of a nosepad of the frame headwear relative to the frame headwear to align a pupil center of an eye of the user with a landmark of a lens placed in front of the eye.

12. Method for providing an eyewear to a user according to claim 10 or 11, wherein the eyewear comprises a set of at least two lens holders, the method further comprising:
- providing another lens holder of the set;
- switching the lens holder with the other lens holder;
- for each lens of the other lens holder, moving the position of the lens relative to the other lens holder to adjust the position of the lens of the other lens holder to the pupillary distance of the user.

13. Method for providing an eyewear to a user according to any of the preceding claims 10 to 12, wherein when the user wear spectacles, the method further comprises:
- mounting the frame headwear over the spectacles, and
- switching in and/or out the lens holder over the spectacles.

14. Method for comparing two different lenses, the method comprising at least:
- providing a frame headwear;
- providing a first lens holder and a second lens holder,
- for each lens holder of the first lens holder and a second lens holder:
∘ fastening the lens holder to the frame headwear;
∘ for each lens of the lens holder, moving the position of the lens relative to the lens holder to adjust the position of the lens to the pupillary distance of the user,
- switching from the first lens holder to the second lens holder at least once after adjusting the position of the lens to the pupillary distance of the user.

15. Method for comparing two different lenses according to claim 14, wherein the lenses have different optical designs and/or different optical powers and/or different tint.
